# EUROPÄISCHE PATENTSCHRIFT

(11) **EP 0 925 080 B1**
(45) Veröffentlichungstag und Bekanntmachung des Hinweises auf die Patenterteilung: **03.03.2004**
(21) Anmeldenummer: 98921407.7
(22) Anmeldetag: 31.03.1998
(51) Int. Cl.: A61M 1/10

(54) **INTRAKARDIALE BLUTPUMPE**
INTRACARDIAC BLOOD PUMP
POMPE A SANG INTRACARDIAQUE

(30) Priorität: 02.04.1997 US 832040
(43) Veröffentlichungstag der Anmeldung: 30.06.1999
(73) Patentinhaber: Impella CardioSystems AG, 52074 Aachen (DE)
(72) Erfinder: NIX, Christoph, D-52076 Aachen (DE); SIESS, Thorsten, D-52146 Würselen (DE)
(74) Vertreter: Selting, Günther, Dipl.-Ing.
(86) Internationale Anmeldenummer: PCT/EP1998/001866
(87) Internationale Veröffentlichungsnummer: WO 1998/043688

(56) Entgegenhaltungen:
- EP-A- 0 445 782
- EP-A- 0 659 444
- WO-A-94/09835
- DE-A- 19 613 388
- US-A- 3 995 617
- YAMAZAKI K ET AL: "DEVELOPMENT OF A MINIATURE INTRAVENTRICULAR AXIAL FLOW BLOOD PUMP" ASAIO JOURNAL, Bd. 39, Nr. 3, 1. Juli 1993, Seiten M224-M230, XP000412587
- TRINKL J ET AL: "CONTROL OF ROTARY PULSATILE CARDIAC ASSIST PUMP DRIVEN BY AN ELECTRIC MOTOR" PROCEEDINGS OF THE ANNUAL INTERNATIONAL CONFERENCE OF THE ENGINEERI IN MEDICINE AND BIOLOGY SOCIETY, PARIS, OCT. 29 - NOV. 1, 1992, Bd. VOL. 2, Nr. CONF. 14, 29. Oktober 1992, Seite 367/368 XP000480453 MORUCCI J P;PLONSEY R; COATRIEUX J L; SWAMY LAXMINATAYAN

## Beschreibung

Die Erfindung betrifft eine intrakardiale Blutpumpe, und insbesondere eine Blutpumpe, die vollständig in das Herz eingeführt werden kann, um die natürliche Pumpfunktion des Herzens zu unterstützen oder durch kontinuierlichen Pumpbetrieb zu ersetzen.

Eine Pumpvorrichtung für die Herzunterstützung ist beschrieben in WO94/09835 (Jarvik). Diese Pumpvorrichtung weist zwei voneinandner unabhängige Pumpen auf, die jeweils aus einem Pumpenteil und einem damit starr verbundenen Antriebsteil bestehen. Der Pumpenteil der einen Pumpe wird durch eine apekale Operationsöffnung hindurch derart in den linken Ventrikel eingeführt, daß er aus dem linken Ventrikel heraus in die Aorta fördert. Der andere Pumpenteil wird durch eine weitere Operationsöffnung hindurch in den rechten Ventrikel so eingeführt, daß er aus dem rechten Ventrikel in die Pulmonalarterie hineinfördert. Das System enthält weiterhin einen Kontroll- und Anzeigemodul, der klein genug ist, um sterilisiert und im sterilen Operationsbereich benutzt zu werden. Dieser kann einen Mikroprozessor mit Kontroll- und Überwachungsalgorithmen enthalten, um den Volumenstrom und Druck zu regulieren, oder um den Volumenstrom und Druck, die durch Sensoren gemessen oder durch Vergleich der Messungen von Geschwindigkeit und Energieaufnahme berechnet wurden, einer Datenbasis zuzuführen. Die als Kanülenpumpen bezeichneten Pumpen können mit eingebauten Drucksensoren oder Volumenstrommeßvorrichtungen ausgestattet sein, um örtliche Messungen dieser Parameter im Rahmen des Patientenmanagement durchzuführen.

Der vorliegenden Erfindung liegt die Aufgabe zugrunde, eine intrakardiale Blutpumpe zu schaffen, deren Betriebsverhalten mit einfachen meßtechnischen Mitteln, die wenig zusätzlichen Platz erfordern, feststellbar ist.

Die Lösung dieser Aufgabe erfolgt erfindungsgemäß mit den Merkmalen des Patentanspruchs 1.

Bei der erfindungsgemäßen Blutpumpe wird in Abhängigkeit von dem an der Auslaßseite der Pumpe herrschenden Druck die Drehzahl des Motors gesteuert. Der Druck an der Druckseite der Pumpe ist ein wichtiger Parameter für die Förderleistung und den Betriebspunkt der Pumpe und er kann für die Messung des Volumenstromes, also der Fördermenge pro Zeiteinheit, benutzt werden. Der Motor kann in der Weise gesteuert werden, daß seine Drehzahl in Abhängigkeit von dem Meßergebnis der Druckmeßeinrichtung verändert wird.

Intrakardial im Sinne der vorliegenden Erfindung umfaßt die Herzkammern (Ventrikel), die Vorhöfe und die angrenzenden Gefäßstümpfe.

Die Druckmeßeinrichtung kann darüber hinaus auch für die Feststellung der Position der Pumpe im Herzen benutzt werden.

Zweckmäßigerweise besteht die Druckmeßeinrichtung aus zwei Drucksensoren, von denen einer den Druck auf der Druckseite der Pumpe und der andere den Druck auf der Einlaßseite der Pumpe mißt, wobei die Steuereinheit die Drehzahl des Motors in Abhängigkeit von den Signalen beider Drucksensoren steuert. Mit Hilfe der Drucksensoren wird der Differenzdruck zwischen der Saugseite und der Druckseite der Pumpe ermittelt. Aus dem Differenzdruck der Pumpe und der Drehzahl des Motors kann unter Verwendung eines hydraulischen Pumpenkennfelds der Volumenstrom errechnet werden. Auf diese Weise erhält man mit einer sehr einfachen Messung, bei der die Sensoren sehr wenig Platz beanspruchen, eine Bestimmung des von der Pumpe geförderten Volumenstroms.

Anstelle von zwei Drucksensoren kann auch ein einziger Differenzdrucksensor vorgesehen sein, der die Druckdifferenz zwischen der Auslaßseite und der Einlaßseite der Pumpe mißt. Ein solcher Differenzdrucksensor liefert keinen Absolutwert eines Druckes, sondern lediglich einen Wert für die Druckdifferenz, die die entscheidende Größe für die Ermittlung des Volumenstromes darstellt. Andererseits kann ein solcher Differenzdrucksensor zusätzlich für die Lagebestimmung der Pumpe im Innern des Herzens benutzt werden.

Die Pumpe ist vorzugsweise als intravasale Pumpe ausgebildet, wie sie in der (nicht vorveröffentlichten)

WO97/37696 beschrieben ist. Eine solche intravasale Blutpumpe ist mit einem Katheter verbunden. Sie hat so geringe Abmessungen, daß sie durch ein Blutgefäß hindurch an den Einsatzort geschoben werden kann, oder auch im Blutgefäß betrieben werden kann. Bei einer solchen intravasalen Blutpumpe haben der Pumpenteil und der Antriebsteil im wesentlichen gleichen Durchmesser, der nicht größer ist als etwa 5 - 7 mm, da die Gefäßweite in den Körperrandbereichen maximal etwas mehr als 7 mm beträgt. Die rigide Länge einer solchen Pumpe darf nicht mehr als etwa 35 mm betragen, damit die Pumpe durch die Krümmungen von Blutgefäßen hindurchgeht. Allerdings kann die Pumpe zusätzlich mit einem flexiblen Schlauch verlängert sein, der die effektive Länge der Pumpe vergrößert.

Andererseits besteht die Möglichkeit, die Pumpe operativ über das herznahe Gefäßsystem in das Herz einzuführen. In jedem Fall ist die Pumpe so klein, daß sie in das Herz, einschließlich der Vorhöfe und der angrenzenden Gefäßstümpfe, hineinpaßt und im Herzen betrieben werden kann, ohne daß Teile der Pumpe aus dem Herzen herausragen. Aus dem Herzen oder den Blutgefäßen herausgeführt wird allenfalls der Katheter, der mit der Pumpe verbunden ist. Dieser Katheter enthält nicht nur die Leitungen zum Zuführen elektrischer Energie zu der Pumpe, sondern auch die Signalleitungen, die von den an den Pumpen vorgesehenen Sensoren zu der extrakorporalen Steuereinheit führen.

Im folgenden werden Ausführungsbeispiele der Erfindung unter Bezugnahme auf die Zeichnungen näher erläutert.

Es zeigen:
- Fig. 1: einen schematischen Längsschnitt durch eine Ausführungsform einer intrakardialen Blutpumpe,
- Fig. 2: ein Ausführungsbeispiel der intrakardialen Anwendung zweier intravasaler Pumpen,
- Fig. 3: ein schematisches Diagramm zur Erläuterung eines Pumpsystems,
- Fig. 4: ein Diagramm zur Verdeutlichung der Abhängigkeit des Volumenstromes von der Druckdifferenz zwischen der Saugseite und der Druckseite einer Pumpe,
- Fig. 5: ein Diagramm des zeitlichen Verlaufs verschiedener Drücke im Herzen,
- Fig. 6: einen Längsschnitt durch einen Teil der Blutpumpe mit einem Differenzdrucksensor und
- Fig. 7: ein Diagramm zur Verdeutlichung der Abhängigkeit des Volumenstromes von dem Motorstrom und der Drehzahl.

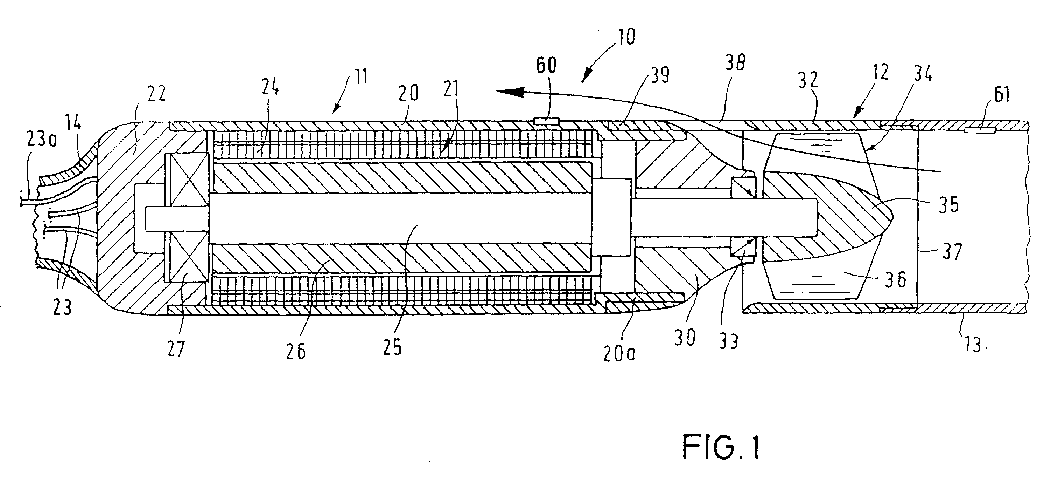

In Fig. 1 ist eine intravasale Blutpumpe 10 dargestellt, also eine Blutpumpe, die durch das Blutgefäßsystem eines Patienten geschoben werden kann, um bis in das Herz hinein vorzudringen. Der Außendurchmesser einer solchen Blutpumpe ist an keiner Stelle größer als 7 mm.

Die Pumpe 10 weist einen Antriebsteil 11 und einen damit starr verbundenen Pumpenteil 12 auf. Der Antriebsteil 11 weist ein langgestrecktes zylindrisches Gehäuse 20 auf, in dem ein Elektromotor 21 untergebracht ist. An dem rückwärtigen Ende ist das Gehäuse 20 mit einer Stirnwand 22 verschlossen, an die sich ein flexibler Katheter 14 abdichtend anschließt. Durch den Katheter 14 verlaufen die elektrischen Kabel 23 zur Stromversorgung und zur Steuerung des Elektromotors 21, und außerdem weitere Kabel 23a, die mit Sensoren der Pumpe 10 verbunden sind.

Der Stator 24 des Motors weist in üblicher Weise zahlreiche umfangsmäßig verteilt angeordnete Wicklungen sowie einen magnetischen Rückschluß in Längsrichtung auf. Er ist mit dem Motorgehäuse 20 fest verbunden. Der Stator 24 umgibt den mit der Motorwelle 25 verbundenen Rotor 26, der aus in Wirkrichtung magnetisierten Permanentmagneten besteht. Die Motorwelle ist am rückwärtigen Ende mit einem Lager 27 im Motorgehäuse bzw. in der Stirnwand 22 gelagert. Die Motorwelle erstreckt sich durch die gesamte Länge des Motorgehäuses 20 und ragt nach vorne aus diesem heraus.

Den vorderen Abschluß des Motorgehäuses bildet ein rohrförmiges stationäres Nabenteil 30, das mit seinem rückwärtigen Ende in einem Ansatz 20a von verringertem Durchmesser des Gehäuses 20 sitzt. Der Außendurchmesser des Nabenteils verjüngt sich zum vorderen Ende hin, wo sich ein Lager 33 zur Lagerung der Motorwelle 25 befindet. Dieses Lager ist zugleich als Wellendichtung ausgebildet.

Die Motorwelle 25 steht aus dem Nabenteil 30 nach vorne vor und trägt dort ein Flügelrad 34 mit einer auf dem Wellenende sitzenden Nabe 35 und davon abstehenden Flügeln 36 oder Pumpenschaufeln, die in bezug auf die Achse des Flügelrades 34 schräggestellt sind. Das Flügelrad 34 ist in einem zylindrischen Pumpengehäuse 32 enthalten, das durch drei umfangsmäßig verteilte Stege 38 mit dem Motorgehäuse 20 verbunden ist. Das Motorgehäuse 20 und das Pumpengehäuse 32 sind durch einen Ring 38 starr miteinander verbunden und haben gleiche Außendurchmesser. Der Durchmesser der Pumpe 10 ist an keiner Stelle größer ist als dieser Außendurchmesser.

Bei einer Rotation des Flügelrades 34 wird Blut durch die Ansaugöffnung 37 des Pumpengehäuses 32 angesaugt und in axialer Richtung im Pumpengehäuse 32 nach hinten getrieben. Durch den ringförmigen Spalt zwischen dem Pumpengehäuse 32 und dem Motorgehäuse 20 strömt das Blut am Nabenteil 30 entlang nach außen, um weiter an dem Motorgehäuse 20 entlangzuströmen. Hierdurch wird der Abtransport der im Antrieb erzeugten Wärme sichergestellt, ohne daß es zur Blutschädigung durch zu hohe Oberflächentemperaturen (über 41 °C) auf dem Motorgehäuse 20 kommt.

Es ist auch möglich, den Pumpenteil 12 für die umgekehrte Förderrichtung auszulegen, wobei das Blut an dem Motorgehäuse entlang angesaugt wird und aus der stirnseitigen Öffnung 37 axial austritt.

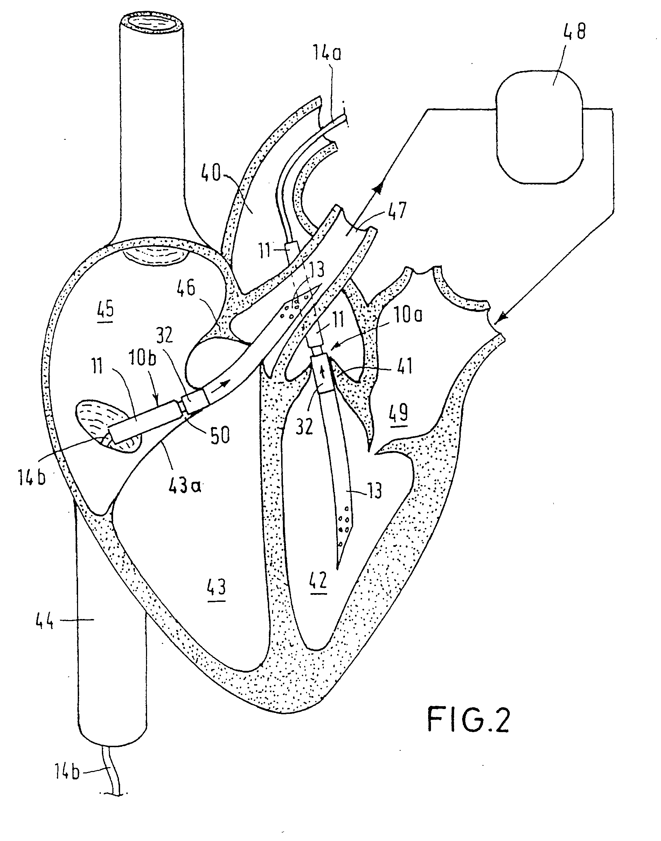

Fig. 2 zeigt ein Ausführungsbeispiel der Verwendung zweier generell einander gleicher Pumpen 10a,10b, die gemäß Fig. 1 ausgebildet sind, in einem Herzen, zur Herzunterstützung oder als Ersatz für die Herzpumpfunktion bei stillgesetztem Herzen. Beide Pumpen 10a,10b sind jeweils mit einem Katheter 14a,14b verbunden. Sie sind percutan verlegt worden, wobei der Katheter 14a der Linksherz-Pumpe 10a durch die Aorta 40 verläuft und ein die Pumpe 10a verlängernder Schlauch 13 durch die Aortenklappe 41 in den linken Ventrikel 42 hinein vorgeschoben ist. Dabei ist der Pumpenteil 12 mit dem mit dem Pumpengehäuse 32 verbundenen flexiblen Schlauch 13 verlängert, der an seinem Ende und/oder in seiner Seitenwand Öffnungen für den Blutzutritt zur Pumpe 10a aufweist. Die Pumpe 10a saugt durch den Schlauch 13 an und fördert das Blut in die Aorta 40, während die Aortenklappe 41 sich von außen gegen das Pumpengehäuse 32 bzw. den Schlauch 13 legt. Im linken Ventrikel 42 wird somit die Pumpe 10a als Linksherz-Pumpe mit axialer Ansaugung betrieben.

Die andere Pumpe 10b wird als Rechtsherzpumpe in Fluidkommunikation mit dem rechten Ventrikel 43 betrieben. Der Katheter 14b führt durch die obere oder untere Hohlvene 44 hindurch in das rechte Atrium 45. Der Schlauch 13 der Pumpe 10b ragt durch die Tricuspidalklappe 43a und die Pulmonalklappe 46 in die Pulmonalarterie 47, von der das Blut zur Oxigenierung zur Lunge 48 fließt. Das oxigenierte Blut fließt dann in den linken Vorhof 49 und von dort in den linken Ventrikel 42.

Die Pumpe 10b saugt durch den radialen Einlaß 50 an und fördert das Blut durch den Schlauch 13 axial in die Pulmonalarterie 47 hinein. Die Pumpe 10b wird also invers zu der Pumpe 10a betrieben.

Beide Pumpen 10a,10b werden in das Herz eingeführt, ohne daß einer der Ventrikel operativ geöffnet werden müßte.

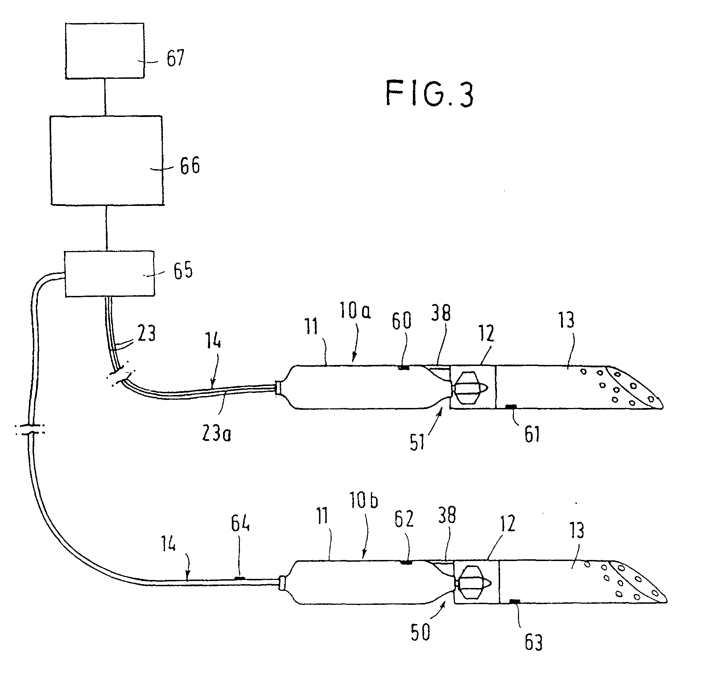

Fig. 3 zeigt eine schematische Darstellung der beiden Pumpen 10a,10b mit verschiedenen Sensoren. Im einzelnen ist an der Außenfläche der Antriebseinheit 11 ein erster Drucksensor 60 vorgesehen, der nahe der radialen Öffnung 51 angeordnet ist, während ein zweiter Drucksensor 61 in der Nähe des Einlasses des Pumpengehäuses angeordnet ist. Die Leitungen 23a der Sensoren sind in die Komponenten der Pumpe integriert und verlaufen gemeinsam mit den Versorgungsleitungen 23 durch den Katheter 14. Der Drucksensor 60 hat seine Sensorfläche an der Außenseite des Motorgehäuses. Dagegen befindet sich die Sensorfläche des Drucksensors 61 an der Innenseite des Schlauchs 13. Ferner kann an dem Antriebsteil ein Temperatursensor angebracht sein, der die Motortemperatur überwacht.

Bei der Pumpe 10b ist ebenfalls ein erster Drucksensor 62 an der Außenseite des Motorgehäuses und ein weiterer Drucksensor 63 an der Innenseite des Schlauchs 13 angeordnet. Die Leitungen dieser Sensoren führen ebenfalls durch den Katheter 14 hindurch. Am Katheter 14 ist ein Sauerstoffsensor 64 angebracht, der Informationen über die Sauerstoffsättigung des Blutes liefert.

Die Versorgungsleitungen 23 und die Leitungen 23a stehen mit einem extrakorporalen Interface 65 in Verbindung. Dieses liefert die Signale der Sensoren an eine Steuereinheit 66, die diese Signale auswertet und in Abhängigkeit davon die Pumpen 10a,10b steuert. Eine Tastatur- und Anzeigevorrichtung 67 ist an die Steuereinheit 66 angeschlossen, um Informationen einzugeben und anzeigen zu können.

Mit den von den Sensoren gelieferten Informationen ist es möglich, die Position einer Pumpe in bezug auf ein externes Umschließungsteil, z.B. eine Herzklappe, festzustellen. Wenn sich Pumpeneinlaß und Pumpenauslaß auf unterschiedlichen Seiten des Umschließungsteils befinden, tritt an den Drucksensoren wegen der unterschiedlichen Druckzustände eine Druckdifferenz auf. Wenn das Herz schlägt, variiert diese Druckdifferenz auch zeitlich. Andererseits sind gleiche Druckmeßwerte ein Anzeichen für eine falsche Pumpenplazierung, da dann beide Drucksensoren denselben Druck messen. Die Auswertung der durch die beiden Drucksensoren gelieferten Daten unter Berücksichtigung des Motorstromes liefert wichtige Informationen über die Anordnung und Operation einer Pumpe. Durch Vergleichen der Druckdifferenz mit dem aktuellen Motorstrom ist es auch möglich, Blockadezustände oder Kavitation festzustellen.

Die Information über den Einlaß- und Auslaßdruck der Pumpe zusammen mit dem Energieverbrauch des Elektromotors liefert wichtige Aussagen über die Funktion der Pumpvorrichtung. Sie liefert auch eine Realzeitangabe über den Volumenstrom und ermöglicht eine Plazierung der Pumpe ohne Röntgenkontrolle oder Ultraschallkontrolle. Ferner kann eine Realzeitüberwachung behinderter Einlaßströmung erfolgen, wie sie z.B. durch Kollabieren des Ventrikels, Thrombenbildung, Okklusion des Schlauchs oder Ansaugen von Herzgewebe hervorgerufen wird. Die Sensoren bieten auch die Möglichkeit, Lagerverschleiß oder Versagen des Motors zu überwachen oder solche Ereignisse vorauszusagen. Ferner kann der Betrieb der Pumpe mit akzeptablen Gesamthämolyseraten über den erforderlichen Benutzungszeitraum und mit dem erforderlichen Volumenstrom von 3,5 bis 5 l/min. aufrechterhalten werden. Die Leistungstrends verschiedener Parameter können über mehrere Betriebsstunden hinweg angezeigt und analysiert werden, wobei Alarmzustände, die ein unverzügliches Eingreifen erfordern, erkannt werden, ohne daß eine permanente Überwachung durch Personen erforderlich ist. Ferner kann das Herz eines Patienten überwacht werden, ohne daß die Pumpe entfernt wird. Mit der Plazierung zweier instrumentierter Pumpen ist es möglich, der Steuereinheit die von einer Pumpe gelieferten örtlichen Informationen zu liefern, um den Betrieb der anderen Pumpe zu steuern und dabei die Leistungsfähigkeit des Systems als Ganzes zu optimieren.

Die Steuereinheit 66 steuert die beiden Pumpen 10a,10b in der Weise, daß jede der beiden Pumpen einen bestimmten Volumenstrom (Blutvolumen pro Zeiteinheit) liefert. Dabei pumpt die Rechtsherz-Pumpe 10b einen bestimmten prozentualen Anteil des Volumenstroms der Linksherz-Pumpe 10a, beispielsweise 90 %. Der Volumenstrom der Rechtsherz-Pumpe ist immer kleiner als derjenige der Linksherz-Pumpe. Primär wird die Pumpenleistung der Linksherz-Pumpe 10a derart geregelt, daß ein bestimmter gewünschter Volumenstrom aufrechterhalten wird. In Abhängigkeit hiervon wird anschließend die Pumpenleistung der Rechtsherz-Pumpe 10b bestimmt. Hierbei handelt es sich um einen Master-Slave-Betrieb, bei dem in der Regel die Linksherz-Pumpe 10a der Master und die Rechtsherz-Pumpe 10b der Slave ist.

Die Pumpen werden von Synchronmotoren angetrieben, wöbei die Steuereinheit 66 die erforderliche Antriebsfrequenz oder Drehzahl n liefert. Der Volumenstrom jeder Pumpe hängt von der Drehzahl n ab.

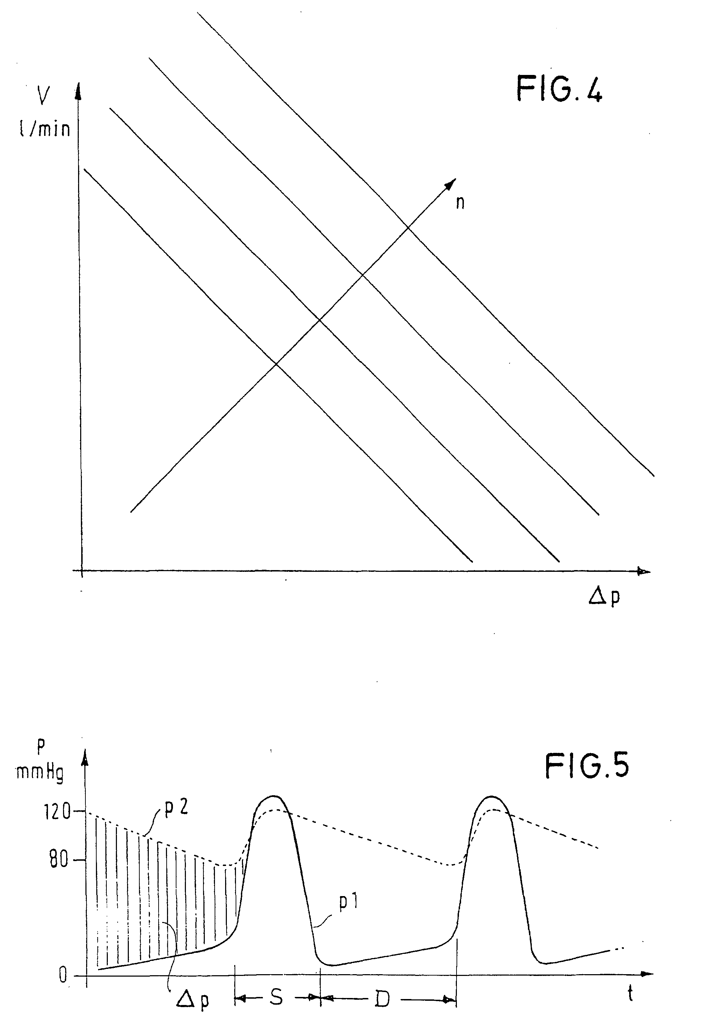

Fig. 4 zeigt den Volumenstrom V einer Pumpe in Abhängigkeit von der Druckdifferenz ΔP zwischen der Saugseite und der Druckseite der Pumpe jeweils für unterschiedliche Drehzahlen n. Jede der parallelen Geraden bezieht sich auf eine bestimmte Drehzahl n. Man erkennt, daß aus der Druckdifferenz ΔP der Volumenstrom V ermittelt werden kann, wenn die Drehzahl n bekannt ist. Der Motor 21 ist ein elektronisch kommutierter Synchronmotor. Da die Drehzahl von der Steuereinheit vorgegeben wird, ist die Drehzahl bekannt. Die Druckdifferenz ΔP wird mit den Sensoren 60 und 61 bzw. 62 und 63 ermittelt. Außerdem werden natürlich auch die Absolutwerte der Drücke gemessen und ausgewertet.

Wenn eine Pumpe durch die radiale Ansaugöffnung 50 bzw. 51 ansaugt und in den Schlauch 13 hinein fördert, ist der Druck an dem schlauchseitigen Drucksensor 61 bzw. 63 größer als an dem saugseitigen Drucksensor 60 bzw. 62. Fördert die Pumpe dagegen in umgekehrter Richtung, d.h. saugt sie durch den Schlauch 13 an, so ist der Druck an dem Drucksensor 60 bzw. 62 höher als derjenige an dem Drucksensor 61 bzw. 63.

Es kann vorkommen, daß eine der Pumpen durch Ansaugen am Herzgewebe oder am Klappenapparat ganz oder teilweise okkludiert wird. In diesem Fall liefern die Drucksensoren abnormale Werte. Dann wird die betreffende Pumpe für eine Zeit in der Drehzahl reduziert, so daß sich das Herzgewebe lösen kann, und anschließend wieder auf die gewünschte Drehzahl erhöht. Wenn der gemessene Absolutdruck zu groß wird, führt die Steuereinheit 66 eine Begrenzung - und ggf. eine Herabsetzung - des Volumenstromes durch, um nachfolgende Organe (Lunge) nicht zu schädigen.

Durch die Druckmessung erfolgt auch eine Wächterfunktion. Der Druck im rechten Ventrikel bzw. in der Pulmonalarterie darf einen bestimmten Wert nicht überschreiten und der Druck im linken Ventrikel bzw. in der Aorta darf einen bestimmten Druck nicht unterschreiten. Werden entsprechende Druckabweichungen festgestellt, wird Alarm gegeben oder nachgeregelt.

In Fig. 5 ist der Druckverlauf p1 im linken Ventrikel des schlagenden Herzens mit den Systolen S und den Diastolen D dargestellt. Man erkennt einen stark pulsierenden Druck, der zwischen den Systolen S stark abfällt. Außerdem ist der Druckverlauf p2 in der Aorta dargestellt. Der Aortendruck pulsiert zwar auch, jedoch in einem viel engeren Druckbereich. Die Druckdifferenz ΔP bestimmt sich zu p2 - pi. Diese Druckdifferenz kann mit den an den Pumpen vorgesehenen Drucksensoren bestimmt werden.

Die Messung der Drücke und Druckdifferenzen ist insbesondere auch für das Einführen der Pumpe in die richtige Position im Herzen wichtig. Das Einführen kann in der Weise geschehen, daß die Pumpe stillgesetzt ist oder mit einer geringen Drehzahl läuft, während das Herz schlägt. Wenn der eine Drucksensor den stark pulsierenden Druckverlauf p1 und der andere den weniger stark pulsierenden Druckverlauf p2 feststellt, ist die Pumpe korrekt plaziert.

Zur Plazierung ist eine Druckmessung jedoch nicht erforderlich. Vielmehr kann die Plazierung auch anhand des Stromverlaufs der Pumpe überwacht werden. Solange sich Einlaß und Auslaß einer Pumpe in demselben Raum befinden, sind beide demselben Druck ausgesetzt. Wird die Pumpe mit einer bestimmten Drehzahl angetrieben, so ist der zeitliche Verlauf des Pumpenstromes konstant. Befinden sich dagegen Auslaß und Einlaß der Pumpe in unterschiedlichen Räumen mit variierenden zeitlichen Druckverläufen, ergibt sich ein nicht glatter, sondern pulsierender Pumpenstrom. Anhand des Pumpenstroms kann somit festgestellt werden, ob die Herzklappe das Pumpengehäuse bzw. den Schlauch ordnungsgemäß umschließt, so daß der Einlaß der Pumpe sich in dem Ventrikel bzw. dem Vorhof und der Auslaß in der Aorta bzw. der Pulmonalarterie befindet.

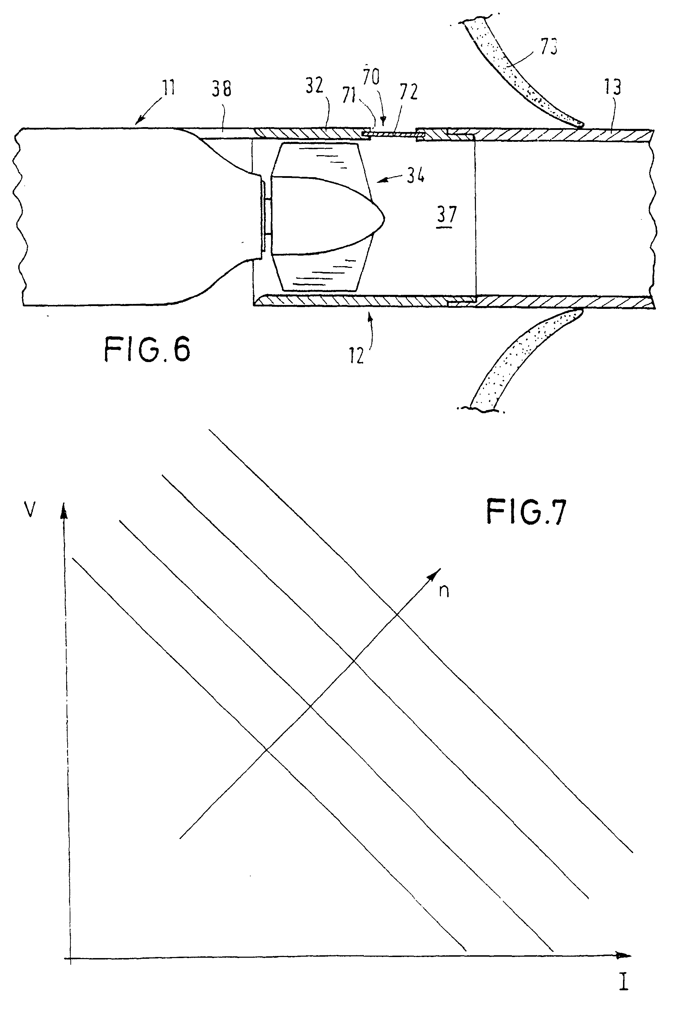

Die bisher beschriebenen Sensoren 61,62 sind Absolutdrucksensoren. Fig. 6 zeigt ein Ausführungsbeispiel mit einem Differenzdrucksensor 70, der in einer Durchbrechung 71 der Wand des Pumpengehäuses 32 angeordnet ist. Der Differenzdrucksensor 70 weist eine flexible Membran 72 auf, die (nicht dargestellte) elektrische Widerstandselemente trägt, deren Widerstand abhängig ist von der Verformung der Membran 72. Wenn die Pumpe durch die Öffnung 37 ansaugt, wirkt auf die Unterseite der Membran 72 der Einlaßdruck der Pumpe. Auf die Außenseite der Membran 72 wirkt der Auslaßdruck der Pumpe. Es sei angenommen, daß eine Herzklappe 73 außen an dem Schlauch 13 anliegt. Wenn der Schlauch 13 durch die Herzklappe 73 hindurchgesteckt ist, entsteht an dem Differenzdrucksensor 70 eine Druckdifferenz. Wenn die Pumpe sich dagegen insgesamt in derselben Kammer befindet, entsteht an dem Drucksensor keine wesentliche Druckdifferenz. Durch Auswertung des Signals des Differenzdrucksensors 70 kann also festgestellt werden, ob die Blutpumpe sich in der korrekten Position befindet.

Für die Lagebestimmung der Blutpumpe im Herzen ist jedoch eine Druckmeßvorrichtung überhaupt nicht erforderlich. Benötigt wird lediglich eine Meßeinrichtung, die ein Informationssignal liefert, welches der Druckdifferenz zwischen der Auslaßseite und der Einlaßseite der Pumpe entspricht. Diese Meßeinrichtung kann aus einer Einrichtung bestehen, die den Motorstrom des die Pumpe treibenden Motors mißt. Das Diagramm von Fig. 7, das ähnlich demjenigen der Fig. 4 ist, zeigt den Volumenstrom V einer Pumpe in Abhängigkeit von dem Strom I des die Pumpe treibenden Motors jeweils für unterschiedliche Drehzahlen n. Jede der parallelen Geraden bezieht sich auf eine bestimmte Drehzahl n. Man erkennt, daß aus der Größe des Stromes I der Volumenstrom V ermittelt werden kann, wenn die Drehzahl n bekannt ist. Auf diese Weise kann durch eine bloße Strommessung der Volumenstrom V ermittelt werden. Darüber hinaus kann mit der Strommessung auch die korrekte Lage der Pumpe im Herzen überprüft werden.

Wenn das Herz schlägt und die Blutpumpe in der Weise plaziert wird wie die Blutpumpe 10a in Fig. 2, entsteht am Einlaß der Blutpumpe der in Fig. 5 mit P1 bezeichnete pulsierende Druck im Ventrikel 42. Am Auslaß der Pumpe entsteht dagegen der in Fig. 5 mit P2 bezeichnete weniger pulsierende Druck. Die Druckdifferenz ΔP ist dann ein pulsierender Wert. Befindet sich andererseits die Blutpumpe noch vollständig in der Aorta, so ist die Druckdifferenz zwischen Einlaß und Auslaß der Pumpe im wesentlichen Null. Gleiches gilt, wenn sich die Blutpumpe vollständig in dem linken Ventrikel 42 befindet. Daher kann durch Feststellung des pulsierenden Differenzdrucks ΔP (Fig. 5) die korrekte Pumpenlage ermittelt werden.

Durch Vergleich der Diagramme der Fign. 4 und 7 erkennt man, daß der vom Motor aufgenommene Strom I für konstante Drehzahlen proportional der Druckdifferenz ΔP ist. Wenn sich somit eine pulsierende Druckdifferenz ΔP ergibt, ist auch der Motorstrom I pulsierend. Somit kann anhand der Pulsation des Motorstromes I das Erreichen der korrekten Pumpenposition im Herzen festgestellt werden. In diesem Fall kann der Verlauf des Stromes I als Informationssignal benutzt werden.

Die Erfindung ermöglicht eine Steuerung des Pumpenbetriebes zur Erzielung eines gewünschten Volumenstromes in Abhängigkeit von dem Ergebnis einer Druckmessung oder dem Ergebnis einer Strommessung. Diese Messungen lassen sich ohne voluminöse Meßvorrichtungen auf sehr einfache Weise durchführen, so daß trotz der kleinformatigen Bauweise der Pumpenbetrieb exakt überwacht werden kann.

## Patentansprüche

1. Intrakardiale Blutpumpe mit einem einen Motor (21) enthaltenden Antriebsteil (11) und einem damit starr verbundenen Pumpenteil (12), wobei der Antriebsteil und der Pumpenteil im wesentlichen gleiche Durchmesser haben und koaxial in axialem Abstand voneinander angeordnet sind, und mit einer Druckmeßeinrichtung (60, 61; 70),
**dadurch gekennzeichnet,**
**daß** die Druckmeßeinrichtung (60,61;70) direkt an der Pumpe derart angeordnet ist, daß sie von dem Druck an der Auslaßseite der Pumpe beeinflußt wird, und daß eine Steuereinheit (66) den Motor (21) in Abhängigkeit von dem Signal der Druckmeßeinrichtung steuert.

2. Blutpumpe nach Anspruch 1, **dadurch gekennzeichnet, daß** die Druckmeßeinrichtung (60,61) einen ersten Drucksensor (60), der den Druck an der Auslaßseite der Pumpe mißt, und einen zweiten Drucksensor (61), der den Druck an der Einlaßseite der Pumpe mißt, aufweist, und daß die Steuereinheit (66) die Drehzahl des Motors (21) in Abhängigkeit von den Signalen beider Drucksensoren (60,61) steuert.

3. Blutpumpe nach Anspruch 2, **dadurch gekennzeichnet, daß** eine Einrichtung vorgesehen ist, die aus den Signalen beider Drucksensoren (60,61) die Druckdifferenz zwischen der Auslaßseite und der Einlaßseite der Pumpe ermittelt und in Abhängigkeit hiervon die Drehzahl des Motors (21) steuert.

4. Blutpumpe nach einem der Ansprüche 1-3, **dadurch gekennzeichnet, daß** die Druckmeßeinrichtung einen Differenzdrucksensor (70) aufweist, der die Druckdifferenz zwischen der Auslaßseite und der Einlaßseite der Pumpe mißt.

5. Blutpumpe nach einem der Ansprüche 1-4, **dadurch gekennzeichnet, daß** die Druckmeßeinrichtung eine Strommeßeinrichtung zur Messung des Motorstromes (I) aufweist und aus dem Motorstrom (I) und der Motordrehzahl (n) die Druckdifferenz zwischen der Auslaßseite und der Einlaßseite des Motors bestimmt.

6. Blutpumpe nach einem der Ansprüche 1-5, **dadurch gekennzeichnet, daß** an dem Antriebsteil (11) ein Temperatursensor vorgesehen ist, der bei Erreichen einer vorgegebenen Temperatur Alarm auslöst.

## Claims

1. An intracardiac blood pump comprising a drive portion (11) with a motor (21), and a pump portion (12) rigidly connected with the drive portion, the drive portion and the pump portion having substantially the same diameter and being arranged coaxially with a mutual axial distance, and comprising a pressure measuring device (60, 61; 70),
**characterized in**
**that** the pressure measuring device (60, 61; 70) is arranged immediately at the pump such that it is influenced by the pressure at the outlet side of the pump, and that a control unit (66) controls the motor (21) as a function of the signal from the pressure measuring device.

2. The blood pump of claim 1, **characterized in that** the pressure measuring device (60, 61) comprises a first pressure sensor (60) measuring the pressure at the outlet side of the pump, and a second pressure sensor (61) measuring the pressure at the inlet side of the pump, and that the control unit (66) controls the rotational speed of the motor (21) as a function of the signals from both pressure sensors (60, 61).

3. The blood pump of claim 2, **characterized in that** means are provided that calculate the differential pressure between the outlet side and the inlet side of the pump from the signals of both pressure sensors (60, 61) and controls the rotational speed of the motor (21) in dependence thereon.

4. The blood pump of one of claims 1 - 3, **characterized in that** the pressure measuring device comprises a differential pressure sensor (70) measuring the differential pressure between the outlet side and the inlet side of the pump.

5. The blood pump of one of claims 1-4, **characterized in that** the pressure measuring device comprises a current measuring device for measuring the motor current (I) and for determining the differential pressure between the outlet side and the inlet side of the motor form the motor current (I) and the rotational speed (n).

6. The blood pump of one of claims 1 - 5, **characterized in that** the drive portion (11) is provided with a temperature sensor that triggers an alarm upon reaching a predetermined temperature.

## Revendications

1. Pompe à sang intracardiaque comprenant une partie motrice (11) renfermant un moteur (21) et une partie pompe (12) qui est reliée de façon rigide à celle-ci, la partie motrice et la partie pompe ayant essentiellement des diamètres identiques et étant disposées coaxialement à distance axiale l'une de l'autre, et comprenant un système de mesure de pression (60, 61 ; 70), **caractérisée en ce que** :
le système de mesure de pression (60, 61 ; 70) est disposé directement sur la pompe de manière à ce qu'il soit influencé par la pression du côté sortie de la pompe, et qu'une unité de commande (66) commande le moteur (21) en fonction du signal du système de mesure de pression.

2. Pompe à sang selon la revendication 1, **caractérisée en ce que** le système de mesure de pression (60, 61) présente un premier capteur de pression (60) qui mesure la pression du côté sortie de la pompe et un second capteur de pression (61) qui mesure la pression du côté entrée de la pompe, et que l'unité de commande (66) commande la vitesse de rotation du moteur (21) en fonction des signaux des deux capteurs de pression (60, 61).

3. Pompe à sang selon la revendication 2, **caractérisée en ce qu'**il est prévu un système qui calcule à partir des signaux des deux capteurs de pression (60, 61) la différence de pression entre le côté sortie et le côté entrée de la pompe et commande en fonction de celle-ci la vitesse de rotation du moteur (21).

4. Pompe à sang selon l'une quelconque des revendications 1 à 3, **caractérisée en ce que** le système de mesure de pression présente un capteur de pression différentielle (70) qui mesure la différence de pression entre le côté sortie et le côté entrée de la pompe.

5. Pompe à sang selon l'une quelconque des revendications 1 à 4, **caractérisée en ce que** le système de mesure de pression présente un système de mesure de courant pour la mesure du courant de moteur (I) et détermine à partir du courant de moteur (I) et de la rotation du moteur (n) la différence de pression entre le côté sortie et le côté entrée du moteur.

6. Pompe à sang selon l'une quelconque des revendications 1 à 5, **caractérisée en ce qu'**il est prévu au niveau de la partie motrice (11) un capteur de température qui déclenche une alarme lors de l'atteinte d'une température prédéfinie.
